(19) Europäisches Patentamt
European Patent Office
Office européen des brevets

(11) **EP 1 698 323 A2**

(12) **DEMANDE DE BREVET EUROPEEN**

(43) Date de publication:
**06.09.2006 Bulletin 2006/36**

(51) Int Cl.:
***A61K 8/37*** *(2006.01)* ***A61K 8/891*** *(2006.01)*
***A61Q 1/12*** *(2006.01)*

(21) Numéro de dépôt: **06300099.6**

(22) Date de dépôt: **02.02.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **04.02.2005 FR 0550342**

(71) Demandeur: **L'ORÉAL**
**75008 Paris (FR)**

(72) Inventeurs:
- **Themens, Agnès**
**92340, Bourg la Reine (FR)**
- **Leuridan, Maïtena**
**75005, Paris (FR)**

(74) Mandataire: **Le Coupanec, Pascale A.M.P. et al**
**Nony & Associés,**
**3 rue de Penthièvre**
**75008 Paris (FR)**

# (54) Composition cosmétique à texture fine

(57) La présente invention concerne une composition cosmétique pulvérulente, caractérisée en ce qu'elle comprend, dans un milieu physiologiquement acceptable, une quantité efficace à titre de liant d'au moins un ester court et d'au moins un dérivé siliconé, la teneur massique en ester(s) court(s) étant supérieure à la teneur massique en dérivé(s) siliconé(s).

EP 1 698 323 A2

## Description

La présente invention concerne une composition cosmétique pulvérulente pour le maquillage de la peau, ainsi qu'un procédé utile pour la préparer.

Une part importante des compositions cosmétiques, et notamment de maquillage telles que les fards à joues, les fards à paupières, et les fonds de teint pour le visage, se présentent sous un aspect pulvérulent. Il s'agit principalement de compositions classiquement qualifiées de "poudres compactes" ou encore de "poudres libres", constituées pour l'essentiel d'un mélange de poudres, colorées ou non, et d'un liant généralement gras comprenant notamment des huiles. Ces poudres sont généralement appliquées sur la peau à l'aide d'un applicateur comme par exemple une éponge, une houppette ou un pinceau.

Outre un effet esthétique en terme de maquillage, l'utilisateur attend généralement de l'utilisation de tels produits d'une part un confort de port et d'application et d'autre part une bonne tenue au cours du temps. Les propriétés de confort de port et d'application se traduisent notamment par des qualités telles que douceur, fluidité, aptitude à glisser et à se fondre sur la peau, et par une texture très fine et crémeuse. Les propriétés de tenue au cours du temps se traduisent notamment par une conservation de bonnes propriétés d'obtention de tenue du maquillage dans le temps. Or, pour les raisons évoquées ci-après, la conciliation de ces deux aspects, confort de port et d'application et tenue au cours du temps, dans une même composition n'est pas totalement optimisée.

En effet, l'aspect fluidité et/ou douceur à l'application est généralement lié à la nature et la quantité du liant retenu. Une composition doit contenir une quantité suffisante en liant pour lui garantir un aspect homogène, pour lui conférer une bonne aptitude à l'étalement lors de son application, pour prévenir l'altération du maquillage au cours du temps, et en outre dans le cas particulier des poudres compactes, pour leur garantir une bonne aptitude au délitage et prévenir leur fragmentation susceptible d'être provoquée notamment par des chocs . Or, dans certaines circonstances, la nature du liant et cette quantité suffisante en liant peuvent s'avérer par ailleurs préjudiciable en terme de confort d'application et de port.

En conséquence, il demeure à ce jour un besoin pour des compositions cosmétiques pulvérulentes, notamment destinées au maquillage de la peau, qui donnent pleinement satisfaction en terme de propriétés de confort d'application et de port et de tenue dans le temps. Plus particulièrement, il existe un besoin pour un liant permettant d'obtenir des compositions donnant satisfaction en ces termes.

La présente invention a précisément pour objet de satisfaire aux besoins précédemment évoqués.

En conséquence, selon l'un de ses aspects, l'invention concerne une composition cosmétique pulvérulente, notamment pour le maquillage de la peau, caractérisée en ce qu'elle comprend, dans un milieu physiologiquement acceptable, une quantité efficace à titre de liant d'au moins un ester court et d'au moins un dérivé siliconé, la teneur massique en ester(s) court(s) étant supérieure à la teneur massique en dérivé(s) siliconé(s).

Selon un autre de ses aspects, l'invention porte sur une composition cosmétique pulvérulente, notamment pour le maquillage de la peau, caractérisée en ce qu'elle comprend, dans un milieu physiologiquement acceptable, une quantité efficace à titre de liant d'au moins un ester court et d'au moins un dérivé siliconé, ladite quantité étant inférieure à 8 % en poids, par rapport au poids total de la composition.

Selon un autre de ses aspects, l'invention concerne également une composition cosmétique pulvérulente, notamment pour le maquillage de la peau, caractérisée en ce qu'elle comprend, dans un milieu physiologiquement acceptable au moins:

- une quantité efficace à titre de liant d'au moins un ester court et
- au moins 15 % en poids d'au moins une charge organique sphérique par rapport au poids total de la composition.

Selon un autre de ses aspects, l'invention concerne une composition cosmétique compactée comprenant, dans un milieu physiologiquement acceptable au moins :

- une phase pulvérulente,
- une quantité efficace à titre de liant d'au moins un ester court, ladite composition possédant après décompactage, une granulométrie D 50 exprimée en volume inférieure à 12 $\mu$m et une polydispersité inférieure à 2.

Les inventeurs ont en outre découvert que des conditions spécifiques de mise en oeuvre d'un tel liant pour la préparation d'une composition cosmétique pulvérulente pouvaient améliorer les qualités attendues au niveau de celle-ci et notamment en terme colorimétrique.

Selon un autre de ses aspects, l'invention concerne une composition cosmétique pulvérulente comprenant à titre de liant au moins un ester court, caractérisée en ce qu'elle est obtenue par micronisation, notamment par jet d'air, de l'ensemble de ses composants, ledit ester court étant ajouté, à l'issue de ladite étape de micronisation sous agitation mécanique, à raison d'au moins 90 % en poids par rapport à son poids total dans ladite composition.

Selon une variante de l'invention, le liant peut comprendre, outre un ester court conforme à l'invention, au moins un dérivé siliconé tel que défini ci-après.

Selon un autre aspect, l'invention concerne un procédé de préparation d'une poudre pulvérulente composée d'au moins une charge pulvérulente et d'une quantité efficace à titre de liant d'au moins un ester court, caractérisé en ce qu'il comprend au moins les étapes consistant à :

- microniser par jet d'air l'ensemble des composants de ladite charge pulvérulente en présence, le cas échéant, de moins de 10 % en poids dudit ester court par rapport à son poids total dans ladite composition et
- mélanger la charge pulvérulente micronisée obtenue à l'issue de l'étape précédente avec au moins 90 % en poids dudit ester court par rapport à son poids total dans ladite composition.

Selon une variante de réalisation, l'ester court est incorporé intégralement à l'issue de l'étape de micronisation. En quel cas, le liant susceptible d'être introduit lors de l'étape de micronisation peut être le dérivé siliconé utilisé conjointement avec l'ester court à titre de liant dans la composition.

En fait, l'introduction de liant lors de l'étape de micronisation est principalement à privilégier pour des questions de sécurité.

Un tel procédé est notamment avantageux dans la mesure où il permet d'optimiser l'effet de saturation en couleur procurée par les matières colorantes au sein de la composition.

Le procédé selon l'invention a en outre pour avantage de conférer à la composition une granulométrie plus faible et plus régulière. Cette granulométrie se traduit pour l'utilisateur en des qualités de toucher de la composition améliorées, en particulier une augmentation de la douceur et de la sensation crémeuse lors du toucher et lors de l'application de la composition. Le compactage de la composition ainsi que l'aspect de la coupelle sont en outre améliorés et la composition ainsi obtenue est plus homogène et possède une texture plus fine.

Selon un autre de ses aspects, l'invention a pour objet un procédé de maquillage d'une matière kératinique, notamment de la peau, comprenant l'application sur la surface à maquiller d'une composition conforme à l'invention.

Comme précisé précédemment, l'invention repose notamment sur l'utilisation, à titre de liant, d'au moins un ester court.

Au sens de l'invention, "quantité efficace à titre de liant" désigne la quantité nécessaire et suffisante en ester (s) court(s), le cas échéant associé(s) à au moins un dérivé siliconé pour assurer, dans le cas des poudres compactes, la cohésion de l'ensemble des particules, et, dans le cas des poudres libres, un aspect homogène, fluidifié et non propice à la formation d'agrégats de particules.

La composition peut contenir de 0,5 à 100 % de liant et, selon un aspect de l'invention, contenir plus particulièrement de 1 à 50 %, notamment de 2 à 15 %, en particulier de 3 à 11 %, encore plus particulièrement de 4 à 9 %, par exemple de 4,5 à 8 %, voire de 5 à 7 % en poids de liant par rapport au poids total de la composition.

Lorsque le liant comprend, à titre d'unique composé, un ou plusieurs ester(s) court(s), les pourcentages précités s'appliquent à la quantité en ester(s) court(s).

**<u>I - ESTER COURT</u>**

Au sens de l'invention, "ester court" désigne les esters des acides monocarboxyliques avec les monoalcools et polyalcools.

Les esters conformes à l'invention peuvent être des monoesters, des diesters ou des polyesters et sont plus particulièrement des monoesters.

Avantageusement, ledit ester répond à la formule (I) suivante :

$$R_1\text{-CO-O-}R_2 \text{ (I)}$$

où $R_1$ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

$R_2$ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par "éventuellement substitué", on entend que $R_1$ et ou $R_2$ peuvent porter un ou plusieurs substituants choisis, par exemple, parmi les groupements comprenant un ou plusieurs hétéroatomes choisi parmi O, N et S, tels que amino, amine, alcoxy et hydroxyle.

De préférence, le nombre total d'atomes de carbone de $R_1 + R_2$ est supérieur ou égal à 9.

Plus particulièrement, les groupements $R_1$ et $R_2$ sont tels que l'ester correspondant est non volatile.

$R_1$ peut représenter le reste d'un acide gras, de préférence supérieur, linéaire ou de préférence ramifié comprenant de 1 à 40 et mieux de 7 à 19 atomes de carbone et $R_2$ peut représenter une chaîne hydrocarbonée linéaire ou de préférence ramifiée contenant de 1 à 40, de préférence de 3 à 30 et mieux de 3 à 20 atomes de carbone. De nouveau, de préférence, le nombre d'atomes de carbone de $R_1$ + $R_2$ supérieur ou égal à 9.

Des exemples des groupes $R_1$ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, éléostéarique, arachidonique, érucique, et de leurs mélanges.

Des exemples d'esters utilisables dans le liant des compositions de l'invention sont, par exemple, l'huile de purcellin (octanoate de cétostéaryle), le néopentanoate d'isodécyle, l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Plus particulièrement, les esters courts selon l'invention sont en $C_9$ à $C_{40}$ et notamment de $C_{10}$ à $C_{25}$ et plus particulièrement en $C_{12}$ à $C_{20}$.

Avantageusement, les esters sont choisis parmi les composés de la formule (I) ci-dessus, dans laquelle $R_1$ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et $R_2$ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, $R_1$ est un groupe alkyle ramifié non substitué de 4 à 14 atomes de carbone, de préférence de 8 à 10 atomes de carbone et $R_2$ est un groupe alkyle ramifié non substitué de 5 à 15 atomes de carbone, de préférence de 9 à 11 atomes de carbone. L'ester court sera choisi, de préférence, parmi les composés suivants :

- le néopentanoate d'isodécyle,
- l'isononanoate d'isononyle,
- l'octanoate de cétostéaryle,
- le myristate d'isopropyle,
- le palmitate d'éthyl-2-hexyle,
- le stéarate d'octyl 2-dodécyle,
- l'érucate d'octyl 2-dodécyle,
- l'isostéarate d'isostéaryle, et leurs mélanges.

Le néopentanoate d'isodécyle s'avère tout particulièrement avantageux pour obtenir des compositions présentant des qualités visuelles et tactiles très satisfaisantes.

Comme précisé ci-dessus, les inventeurs ont constaté que l'utilisation d'au moins un ester court à titre de liant s'avère particulièrement avantageuse pour garantir à des compositions pulvérulentes, une faible granulométrie. Dans le cas particulier des poudres compactes, cette granulométrie est avantageusement préservée même après décompactage.

Ainsi, dans le cas particulier d'une poudre compacte, cette composition peut posséder, après décompactage, une granulométrie (D50) variant de 6 à 12 $\mu$m, notamment variant de 7 à 10,5 $\mu$m, avec (D50) correspondant à la taille de particule définie de telle sorte que 50 % en volume des particules ont une taille inférieure à (D50).

La polydispersité peut être inférieure à 2, notamment inférieure à 1,8, en particulier inférieure à 1,6.

Par polydispersité, on entend la valeur du rapport $\frac{(D90)-(D10)}{(D50)}$, avec (D90) correspondant à la taille de particule définie de telle sorte que 90 % en volume des particules ont une taille inférieure à (D90), (D10) correspondant à la taille de particule définie de telle sorte que 10 % en volume des particules ont une taille inférieure à (D10) et (D50) tel que défini précédemment.

La taille moyenne en volume peut être appréciée par un granulomètre laser, tel que par exemple le Mastersizer 2000 de Malvern ou le BI90 + de Broockhaven Instrument Corporation.

Cette mesure de granulométrie peut notamment être réalisée selon le protocole suivant :

Le produit compacté est cassé à l'aide d'une spatule au dessus d'un tamis possédant une maille de 250$\mu$. Les agrégats de poudre obtenus sont ensuite tamisés. Le tout est récupéré et les caractéristiques granulométriques déterminées avec le granulomètre Malvern.

Comme précisé précédemment, l'ester court est présent dans la composition en une quantité suffisante pour y assurer une fonction de liant. Pour des raisons évidentes, cette quantité est susceptible de varier significativement selon la nature de l'ester court.

A titre illustratif et non limitatif, le liant de la composition peut comprendre avantageusement de 2 à 100 % en poids, de préférence de 50 à 100 % en poids, de préférence encore de 50 à 75 % poids d'ester(s) court(s).

**II - DERIVE SILICONE**

Selon un aspect particulier de l'invention, le liant associe à au moins un ester court au moins un dérivé siliconé.

Avantageusement, le rapport pondéral ester(s) court(s)/dérivé(s) siliconé(s) peut être supérieur ou égal à 1, notamment supérieur à 1,5, plus particulièrement encore supérieur à 2.

Le terme « dérivé siliconé » englobe l'ensemble des composés contenant du silicone et appartenant respectivement à la famille des huiles siliconées, à la famille des résines siliconées, à la famille des cires siliconées et à la famille des gommes siliconées.

Dans une première variante de l'invention, le liant peut associer à au moins un ester court, au moins deux dérivés siliconés appartenant à la même famille, par exemple, deux huiles siliconées.

Dans une autre variante de l'invention, le liant peut associer à au moins un ester court, au moins deux dérivés siliconés appartenant à deux familles distinctes, par exemple, une huile siliconée et une résine siliconée.

**1) Huile siliconée**

Selon une variante de l'invention, le liant comprend, outre au moins un ester court, au moins une huile siliconée.

L'huile siliconée peut être choisie parmi les huiles siliconées volatiles linéaires ou cycliques, telles que les polydiméthyl siloxanes (PDMS) linéaires ou cycliques ayant de 3 à 7 atomes de silicium.

A titre d'exemple de telles huiles, on peut citer les composés cités dans le tableau 1 ci-dessous.

Les huiles siliconées non volatiles peuvent être des polydiméthylsiloxanes, des polyalkylméthylsiloxanes, des diméthicone copolyols, des alkylméthicone copolyols, la cétyldiméthicone, des silicones à groupes alkylglycéryl éthers, des silicones à groupes amines latéraux et le dilauroyltriméthylol propane siloxysilicate. Les groupements alkyle de ces huiles ont notamment de 2 à 24 atomes de carbone.

Les huiles siliconées non volatiles utilisables dans le liant de l'invention, qui contient au moins un ester court, peuvent être en particulier les polydiméthylsiloxanes (PDMS) non volatils, linéaires, liquides à température ambiante ; les polydiméthylsiloxanes comportant des groupements alkyle, alcoxy ou phényle, pendants et/ou en bout de chaîne siliconée, groupements ayant chacun de 2 à 24 atomes de carbone ; les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, les silicones fluorées à groupement(s) pendant(s) ou en bout de chaîne ayant de 1 à 12 atomes de carbone dont tout ou partie des atomes d'hydrogène est substitué par des atomes de fluor, les diméthiconols et leurs mélanges.

Au sens de l'invention, les huiles volatiles présentent à température ambiante (25°C) et pression atmosphérique (760 mm de Hg) une pression de vapeur allant de 0,02 mm à 300 mm de Hg (2,66 Pa à 40 000 Pa) et mieux allant de 0,1 à 90 mm de Hg (13 Pa à 12 000 Pa). Les huiles non volatiles correspondent alors à une pression de vapeur inférieure à 0,02 mm de Hg (2,66 Pa).

Les huiles siliconées ont une viscosité choisie avantageusement dans la gamme allant de 5 à 800 000 cSt à 25°C, de préférence de 10 à 500 000 cSt, et mieux de 10 à 5 000 cSt.

Tableau 1

| Composé | Point éclair (°C) | Viscosité (cSt) |
|---|---|---|
| Octyltriméthicone | 93 | 1,2 |
| Hexyltriméthicone | 79 | 1,2 |
| Décaméthyl cyclopentasiloxane (cyclopentasiloxane ou D5) | 72 | 4,2 |
| Octaméthylcyclo tétrasiloxane (cyclotétradiméthyl siloxane ou D4) | 55 | 2,5 |
| Dodécaméthylcyclo hexasiloxane (D6) | 93 | 7 |
| Décaméthyltétrasiloxane (L4) | 63 | 1,7 |
| KF 96 A de Shin Etsu | 94 | 6 |
| PDMS (polydiméthylsiloxane) DC 200 | 56 | 1,5 |
| (1,5 cSt) de Dow Corning PDMS DC 200 (2 cSt) de Dow Corning | 87 | 2 |
| PDMS DC 200 (5 cSt) de Dow Corning | 134 | 5 |

(suite)

| Composé | Point éclair (°C) | Viscosité (cSt) |
|---|---|---|
| PDMS DC 200 (3 cSt) de Dow Corning | 102 | 3 |

En d'autres termes, la(les) huile(s) siliconée(s) volatile(s) peut(vent) être choisie(s), par exemple, dans le groupe constitué par les composés du tableau 1, l'heptaméthyloctyltrisiloxane, le dodécaméthylpentasiloxane, le polyméthylcétyldiméthylsiloxane, et leurs mélanges.

L'huile siliconée volatile peut aussi être choisie dans le groupe des huiles siliconées fluorées telles que les silicones à groupes alkyle et perfluoralkyle, les silicones à groupes latéraux oxyéthylénés/oxypropylénés (OE/PP) et à groupes perfluorés, les silicones à groupes latéraux perfluorés ou polyfluorés et à groupes latéraux glycérolés, et les perfluoroalkylméthylphénylsiloxanes, ces huiles ayant une pression de vapeur supérieure ou égale à 0,02 mm Hg.

Selon un aspect de l'invention, le liant contient avantageusement de 0,5 à 98 % d'huile siliconée, de préférence de 1 à 50 % en poids, de préférence encore de 10 à 40 % en poids, mieux de 20 à 30 % en poids.

Conviennent tout particulièrement comme huiles siliconées les polydiméthylsiloxanes qui permettent d'obtenir les caractéristiques souhaitées pour la composition.

Dans une réalisation particulièrement avantageuse, le liant comprend de 50 à 80 % d'ester(s) court(s) et de 20 à 50 % d'huile(s) siliconée(s).

Par exemple, un liant à base de néopentanoate d'isodécyle avec de la polydiméthylsiloxane s'avère tout particulièrement avantageux.

**2) Résines siliconées**

Selon une variante de l'invention le liant comprend, outre au moins un ester court, au moins un dérivé siliconé dont au moins une résine siliconée.

Les résines de silicone utilisables dans le cadre de l'invention sont généralement solubles ou gonflables dans les huiles de silicone. Ces résines sont des polymères de polyorganosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène.

La lettre T représente une unité trifonctionnelle de formule $(CH_3)SiO_{3/2}$, dans laquelle l'atome de silicium est relié à trois atomes d'oxygène.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle $SiO_{4/2}$ dans laquelle l'atome de silicium est lié à quatre atomes d'hydrogène eux mêmes liés au reste du polymère.

Diverses résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :

- les siloxysilicates qui peuvent être des triméthylsiloxysilicate de formule (II) :

$$[(CH_3)_3\text{-i-O}]_x\text{-}(SiO_{4/2})_y \qquad \text{(II)}$$

  (unités MQ) dans laquelle x et y sont des entiers allant de 50 à 80,
- les polysilesquioxanes de formule $(CH_3SiO_{3/2})_{.x}$ (unités T) dans laquelle x est supérieur à 100 et dont au moins un des radicaux méthyle peut être substitué par un groupement R tel que défini plus haut,
- les polyméthylsilsesquioxanes qui sont des polysilsesquioxanes dans lesquels aucun des radicaux méthyle n'est substitué par un autre groupement. De tels polymethylsilsesquioxanes sont décrits dans le document US 5,246,694 dont le contenu est incorporé par référence.

A titre d'exemples de résines polyméthylsilsesquioxanes commercialement disponibles, on peut citer celles qui sont commercialisées :

- par la société WACKER sous la référence RESIN MK tels que la BELSIL PMS MK : polymère comprenant des unités répétitives $CH_3SiO_{3/2}$ (unités T), pouvant aussi comprendre jusqu'à 1% en poids d'unités $(CH_3)_2SiO_{2/2}$ (unités D) et présentant un poids moléculaire moyen d'environ 10000,
- par la société SHIN-ETSU sous les références KR-220L qui sont composé d'unités T de formule $CH_3SiO_{3/2}$ et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88 % d'unités T et 12 % d'unités dimethyl D et ont des groupes terminaux Si-OH.

Comme résines siloxysilicates, on peut citer les résines trimethylsiloxysilicate (TMS) éventuellement sous forme de poudres. De telles résines sont commercialisées sous la référence SR1000 par la société GENERAL ELECTRIC ou sous la référence TMS 803 par la société WACKER. On peut encore citer les résines triméthylsiloxysilicate commercialisées dans un solvant tel que la cyclomethicone, vendues sous la dénomination "KF-7312J" par la société SHIN-ETSU, "DC 749", "DC 593" par la société DOW CORNING.

Parmi les résines siliconées, la triméthylsiloxysilicate est particulièrement avantageuse dans le cadre de l'invention.

Dans une réalisation particulièrement avantageuse, le liant comprend entre 50 et 80 % en masse d'esters courts, entre 20 et 50 % d'une huile siliconée et entre 0 et 30 % d'une résine de silicone.

Dans une autre réalisation conforme à l'invention, le liant comprend entre 60 et 99,95 % d'ester court et de 0,05 à 40 % d'une résine siliconée.

Par exemple, convient tout particulièrement à l'invention, un liant associant à du néopentanoate d'isodécyle, de la polydiméthylsiloxane et du triméthylsiloxy silicate.

**3) Cires siliconées**

Selon une variante de l'invention le liant comprend, outre au moins un ester court, au moins un dérivé siliconé dont au moins une cire siliconée.

Les cires de silicone utilisables dans le liant gras de la présente invention sont des polysiloxanes substitués solides ou liquides à température ambiante. Ce sont de préférence des fluides ou solides à bas point de fusion. Il s'agit notamment de polysiloxanes linéaires substitués constitués essentiellement (les groupes terminaux mis à part) de motifs de formules III et IV, dans les proportions molaires respectives m et n :

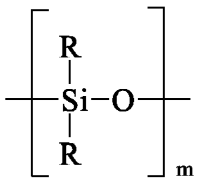

et

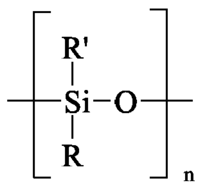

(III) (IV)

dans lesquelles chaque substituant R est un groupement alkyle inférieur (ayant 1 à 6 carbones), chaque R' représente indépendamment un alkyle (linéaire ou ramifié) éventuellement insaturé, ayant 6-30 atomes de carbone, ou bien un groupement -X-R", chaque X représente indépendamment :

- -O-,
- $(CH_2)_a$-O-CO,
- $(CH_2)_b$-CO-O,
- a et b représentent indépendamment des nombres pouvant varier de 0 à 6, et chaque R" représente indépendamment un groupement alkyle, éventuellement insaturé, ayant 6 à 30 atomes de carbone,
- m est un nombre pouvant varier de 0 à 400, et en particulier de 0 à 100,
- n est un nombre pouvant varier de 1 à 200, et en particulier de 1 à 100,
- la somme (m + n) étant inférieure à 400, et en particulier inférieure ou égale à 100.

Ces cires de silicones sont connues ou peuvent être préparées selon les méthodes connues. Parmi les cires de silicones commerciales de ce type, on peut citer nomment celles vendues sous les dénominations Abilwax 9800® , 9801® ou 9810® (GOLDSCHMIDT), KF910® et KF7002® (SHIN ETSU), ou 176-1118-3® et 176-11481® (GENERAL ELECTRIC).

Parmi ces cires siliconées, la polyméthylcétyl diméthylsiloxane convient particulièrement dans le cadre de l'invention.

Les cires de silicone utilisables peuvent également être choisies parmi les composés de formule V :

$$R_1\text{-Si}(CH_3)_2\text{-O-}]_z\text{-Si}(CH_3)_2\text{-}R_2 \qquad (V)$$

dans laquelle

- R est défini comme précédemment,
- $R_1$ représente un groupement alkyle de 1 à 30 C, un groupement alcoxy de 6 à 30 C ou un groupement de formule :

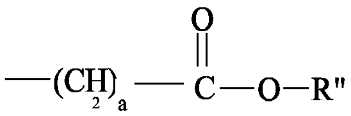 ou 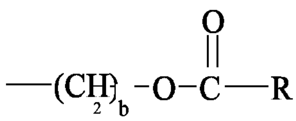

- $R_2$ représente un groupement alkyle de 6 à 30 C, un groupement alcoxy ayant de 6 à 30 C ou un groupement de formule :

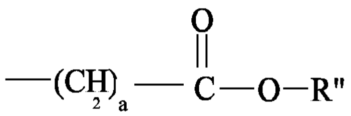 ou 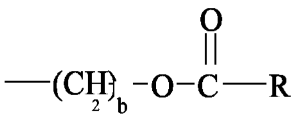

- a et b représentant un nombre de 0 à 6,
- R" étant un alkyle de $C_6$ à $C_{30}$, et
- z est un nombre pouvant varier de 1 à 100.

Parmi les cires de silicone de formule V, qui sont des produits connus ou pouvant être préparés selon les méthodes connues, on citera notamment les produits commerciaux suivants : Abilwax 2428® , 2434® et 2440® (GOLDSCHMIDT), ou VP 1622® et VP 1621® (WACKER).

Dans une réalisation particulièrement avantageuse, le liant comprend de 50 à 80 % en poids d'ester(s) court(s), de 10 à 50 % d'huile(s) siliconée(s), de 0 à 20 % de résine(s) de silicone et de 0 à 20 % de cire(s) de silicone.

Dans une autre réalisation conforme à l'invention, le liant comprend de 60 à 99,95 % d'ester(s) court(s) et de 0,05 à 40 % de cire(s) siliconée(s). Par exemple, convient tout particulièrement à l'invention, un liant associant à du néopentanoate d'isodécyle, de la polydiméthylsiloxane, du triméthylsiloxy silicate et de la polyméthylcétyl diméthylsiloxane.

**4) Gommes siliconées**

Selon une variante de l'invention le liant comprend, outre au moins un ester court, au moins un dérivé siliconé dont au moins une gomme siliconée.

Les gommes de silicone utilisables, conformément à la présente invention, sont des polysiloxanes ayant des masses moléculaires élevées, pouvant aller par exemple de 200 000 à 1 000 000. Elles sont utilisées seules ou en mélange dans un solvant. Ce solvant peut être choisi notamment parmi les huiles polydiméthylsiloxanes (PDMS) et les huiles polyphénylméthylsiloxanes (PPMS). Il s'agit également de produits connus et commercialisés ou pouvant être préparés selon les méthodes connues. On peut citer plus particulièrement les gommes de silicone suivantes : polydiméthylsiloxane/méthylvinylsiloxane, polydiméthylsiloxane/diphénylsiloxane, polydiméthylsiloxane/phénylméthylsiloxane et polydiméthylsiloxane/diphénylsiloxane/méthylvinylsiloxane. Parmi les gommes de silicone commerciales, on peut citer celles vendues sous les dénominations SE30 (GENERAL ELECTRIC), TP232 (UNION CARBIDE), Q2-1403 (DOW CORNING), ou la série VISCASIL (GENERAL ELECTRIC).

**III- PHASE PULVERULENTE**

La phase pulvérulente, généralement présente dans la composition selon l'invention, comporte au moins une charge inorganique ou organique sphérique ou lamellaire, associée généralement à au moins une matière colorante inorganique, notamment de type pigment.

Les compositions selon l'invention peuvent contenir de 0,5 à 99,5 % en poids de phase pulvérulente, et selon un aspect de l'invention peuvent contenir plus particulièrement de 75 à 98 % de phase pulvérulente, notamment de 85 à 97 %, en particulier de 90 à 96 %, voire de 93 à 95 % en poids de charge pulvérulente.

**1. Charges**

Les charges peuvent être minérales ou organiques. Les charges peuvent être des particules de toute forme, notamment plaquettaires, sphériques ou oblongues, quelle que soit leur forme cristallographique (par exemple feuillet, cubique, hexagonale, orthorhombique, etc...).

Parmi les charges utilisables dans les compositions selon l'invention, on peut notamment citer le talc, le mica naturel ou synthétique, la silice, le kaolin, les poudres de polyamide (Nylon® ), de poly-β-alanine et de polyéthylène, les poudres de polymères de tétrafluoroéthylène (Téflon® ), la lauroyl-lysine, l'amidon, le nitrure de bore, les poudres de polymères d'acide acrylique,

- les microbilles de résine de silicone (TOSPEARLS® de TOSHIBA, par exemple),
- les oxychlorures de bismuth, le carbonate de calcium précipité, le carbonate et l'hydro-carbonate de magnésium, l'hydroxyapatite.
- les microsphères de silice creuses, (telles que les 'SILICA BEADS SB 700/HA® ' ou 'SILICA BEADS SB 700® ' de la société MAPRECOS, les 'SUNSPHERES H-33® ' et les 'SUNSPHERES H-51® ' de la société ASAHI GLASS),
- les microsphères de polymères acryliques, (telles que celles en copolymère d'acrylate réticulé 'POLYTRAP 6603 ADSORBER® ' de la société RP SCHERRER, et celles de polyméthacrylate de méthyle 'MICROPEARL M 100® ' de la société SEPPIC),
- les poudres de polyuréthane (telle que la poudre de copolymère de diisocyanate d'hexaméthylène et de triméthylol hexyl lactone vendue sous la dénomination « PLASTIC POWDER D-400® » par la société TOSHIKI),
- les microcapsules de verre ou de céramique,
- les savons métalliques dérivés d'acides organiques carboxyliques ayant de 8 à 22 atomes de carbone, notamment de 12 à 18 atomes de carbone, par exemple le stéarate de zinc, de magnésium ou de lithium, le laurate de zinc, le myristate de magnésium,
- les microcapsules de polymères ou de copolymères d'acrylate ou de méthacrylate de méthyle, ou encore de copolymères de chlorure de vinylidène et d'acrylonitrile, comme 1' « EXPANCEL® » de la société EXPANCEL ;
- les poudres d'organopolysiloxane réticulés élastomères, telles que celles vendues sous la dénomination « TREFIL POWDER E-506C» par la société DOW CORNING,
- les poudres d'organopolysiloxane réticulé élastomère enrobée de résine de silicone, telles que celles vendues sous les dénomination "KSP-100", "KSP-101 ", "KSP-102", "KSP-103", KSP-104", "KSP-105" par la société SHIN ETSU, et
- leurs mélanges.

Selon un mode de réalisation de l'invention, la composition contient au moins une charge organique sphérique notamment choisie parmi celles citées précédemment. Il peut notamment s'agir de poudres de polyamides (Nylon® ) (Orgasol® de chez Atochem), de poudres de polyéthylène, de poudres de polymères de tétrafluoroéthylène (Téflon® ), d'amidon, de microsphères polymériques telles que celles de chlorure de polyvinylidène/acrylonitrile comme l'Expancel® (Nobel Industrie), de copolymères d'acide acrylique (Polytrap® de la société Dow Corning), de microbilles de résine de silicone (Tospearls® de Toshiba, par exemple), et d'organopolysiloxanes élastomères.

**2. Matière colorante inorganique ou organique**

La phase pulvérulente de la composition selon l'invention peut avantageusement contenir au moins une matière colorante pulvérulente pouvant être choisie parmi les pigments et les nacres habituellement utilisés dans les compositions cosmétiques et/ou dermatologiques.

Les pigments peuvent être blancs ou colorés, minéraux et/ou organiques, enrobés ou non. On peut notamment citer parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut notamment citer le noir de carbone, les pigments de type D &

C, et les laques à base de carmin de cochenille, de baryum, strontium, calcium, aluminium.

Les nacres peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La matière colorante pulvérulente peut généralement être présente dans la composition en une teneur variant de 0,5 à 35 % en poids, notamment de 1 à 20 % en poids, et en particulier de 3 à 15 % en poids par rapport au poids total de la composition.

## IV- MILIEU PHYSIOLOGIQUEMENT ACCEPTABLE

Par "milieu physiologiquement acceptable", on désigne un milieu non toxique et susceptible d'être appliqué sur les matières kératiniques et particulièrement sur la peau d'êtres humains. Le milieu physiologiquement acceptable est généralement adapté à la nature de la peau sur laquelle doit être appliquée la composition ainsi qu'à la forme sous laquelle la composition est destinée à être conditionnée, notamment sous une forme de poudre compacte ou libre sous pression atmosphérique.

Ainsi, les compositions selon l'invention peuvent être formulées sous une forme de type poudre libre, compacte.

Outre les composés précités, la composition selon l'invention peut comprendre des dérivés non siliconés tels que les huiles non siliconées et/ou les corps gras solides non siliconés.

Il est entendu que la présence de ces composés dans la composition selon l'invention ne doit pas être préjudiciable à la manifestation des qualités attendues en terme de confort d'application et d'effet esthétique procurés par ailleurs par la présence de l'ester court, éventuellement associé à un dérivé de silicone.

### 1. Huile non siliconée

Les huiles non siliconées volatiles peuvent être choisies dans le groupe des huiles hydrocarbonées et des esters et éthers volatils tels que les hydrocarbures volatils comme l'isododécane et l'isohexadécane, les isoparaffines en $C_8$-$C_{16}$.

L'huile non siliconée volatile peut aussi être choisie parmi les huiles fluorées telles que les perfluoropolyéthers, les perfluoroalcanes comme la perfluorodécaline, les perfluorodamantanes, les monoesters, diesters et triesters de perfluoroalkylphosphates et les huiles esters fluorés.

A titre d'exemple d'huiles non siliconées volatiles utilisables dans la composition de l'invention, on peut citer les composés du tableau 2 qui suit.

Tableau 2

| Composé | Point éclair (°C) |
|---|---|
| Isododécane | 43 |
| Isohexadécane | 102 |
| n-butyléther de propylène glycol | 60 |
| 3-éthoxypropionate d'éthyle | 58 |
| Acétate de méthyléther de propylène glycol | 46 |
| Isopar L (isoparaffine C11-C13) | 62 |
| Isopar H (isoparaffine C11-C12) | 56 |

La composition peut également contenir au moins une huile polaire telle que :

- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel,
- les éthers de synthèse ayant de 10 à 40 atomes de carbone,
- les alcools gras en $C_8$ à $C_{26}$ comme l'alcool oléique ou l'octyldodécanol,

- les acides gras comme l'acide oléique, linoléique ou linolénique, et
- leurs mélanges.

Elle peut encore contenir une ou plusieurs huiles apolaires telles que les hydrocarbures ou fluorocarbures, linéaires ou ramifiés, d'origine synthétique ou minérale, volatils ou non, comme les huiles de paraffine volatiles (telles que les isoparaffines, l'isododécane) ou non volatiles et ses dérivés, la vaseline, les polydécènes, le polyisobutène hydrogéné tel que le parléam, le squalane, et leurs mélanges.

**2. Corps gras solide non siliconé**

Les compositions selon l'invention peuvent en outre comprendre au moins un corps gras solide non siliconé, qui peut être choisi parmi les cires et les composés pâteux.

Par "cire" au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le colorimètre vendu sous la dénomination DSC 30 par la société METLER.

La cire peut également présenter une dureté allant de 0,05 MPa à 15 MPa, et de préférence allant de 6 MPa à 15 MPa. La dureté est déterminée par la mesure de la force en compression mesurée à 20 °C à l'aide du texturomètre vendu sous le dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Les cires peuvent être hydrocarbonées, fluorées et/ou siliconées et être d'origine végétale, minérale, animale et/ou synthétique. En particulier, les cires présentent une température de fusion supérieure à 30 °C et mieux supérieure à 45 °C.

Comme cire utilisable dans la première composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch.

Les compositions peuvent également contenir une cire micronisée, appelée également micro cire.

Comme micro cires pouvant être utilisées dans les compositions selon l'invention, on peut citer notamment les micro cires de carnauba telles que celle commercialisée sous la dénomination de "MicroCare 350®" par la société MICRO POWDERS, les micro cires de cire synthétique telles que celle commercialisée sous la dénomination de "MicroEase 114S®" par la société MICRO POWDERS, les micro cires constituées d'un mélange de cire de carnauba et de cire de polyéthylène telles que celles commercialisées sous les dénominations de "Micro Care 300®" et "310®" par la société MICRO POWDERS, les micro cires constituées d'un mélange de cire de carnauba et de cire synthétique telles que celle commercialisée sous la dénomination "Micro Care 325®" par la société MICRO POWDERS, les micro cires de polyéthylène telles que celles commercialisées sous les dénominations de "Micropoly 200®", "220®", "220L®" et "250S®" par la société MICRO POWDERS et les micro cires de polytétrafluoroéthylène telles que celles commercialisées sous les dénominations de "Microslip 519®" et "519 L®" par la société MICRO POWDERS.

Parmi les micro cires citées précédemment, certaines telles que par exemple la micro cire de carnauba, la micro cire de cire synthétique "MicroEase 114S», ou la micro cire constituée d'un mélange de cire de carnauba et de cire synthétique "MicroCare 325" présentent un point de fusion commençante supérieur ou égal à 45 °C.

Ces compositions peuvent en outre contenir au moins un composé pâteux, qui peut être avantageusement choisi parmi :

- la lanoline et ses dérivés,
- les composés fluorés polymères ou non,
- les polymères vinyliques, notamment :
    - les homopolymères d'oléfines,
    - les copolymères d'oléfines,
    - les homopolymères et copolymères de diènes hydrogénés,
    - les oligomères linéaires ou ramifiés, homo ou copolymères de (méth)acrylates d'alkyles ayant de préférence un groupement alkyle en C8-C30,
    - les oligomères homo et copolymères d'esters vinyliques ayant des groupements alkyles en C8-C30,
    - les oligomères homo et copolymères de vinyléthers ayant des groupements alkyles en C8-C30,

- les polyéthers liposolubles résultant de la polyéthérification entre un ou plusieurs diols en C2-C100, de préférence en C2-C50,
- les esters,
- et leurs mélanges.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol
- le propionate d'arachidyle commercialisé sous la marque Waxenol 801 par Alzo,
- les esters de phytostérol,
- les triglycérides d'acides gras et leurs dérivés,
- les esters de pentaérythritol,
- et leurs mélanges.

Parmi les composés pâteux d'origine végétale, on choisira de préférence un mélange de stérols de soja et de pentaérythritol oxyéthyléné (5 OE) oxypropyléné (5 OP), commercialisé sous la référence Lanolide par la société VEVY.

La composition peut également comprendre d'autres ingrédients (adjuvants) usuellement utilisés en cosmétique tels que par exemple, des agents colorants hydrosolubles ou liposolubles, des conservateurs, des actifs cosmétiques, des agents hydratants, des filtres UV, des épaississants, de l'eau, des tensioactifs, des gélifiants et/ou des parfums.

Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.

La composition selon l'invention peut notamment se présenter sous la forme d'un produit de maquillage de type poudre compact ou libre, en particulier un fard à joues, un fard à paupières, une poudre pour le visage, un fond de teint, un produit anti-cernes, un produit de maquillage du corps, ou bien encore sous la forme d'un produit de soin pour le visage ou d'un produit de soin du corps.

Les exemples suivant illustrent l'invention. Dans ces exemples, les quantités des divers ingrédients sont données en partie en poids.

__**Exemples 1 à 4**__

Préparation d'un fond de teint comprenant en pourcentages massiques :

| | Exemple 1 | Exemple 2 | Exemple 3 | Exemple 4 |
|---|---|---|---|---|
| Mélange P-Hydroxybenzoates de méthyle, éthyle, propyle, butyle isobutyle/phénoxy-2 éthanol (PHENONIP de la société NIPA 5CLARIANT) | 0,60 % | 0,60 % | 0,60 % | 0,60 % |
| Titanium dioxide | 0,00 % | 2,00 % | 2,00 % | 2,00 % |
| Oxyde de fer jaune (CI: 77492) | 1,90 % | 1,90 % | 2,20 % | 1,90 % |
| Oxydes de fer brun, jaune (75/25) (CI : 77491 + 77492) | 0,80 % | 0,80 % | 1,00% | 0,80 % |
| Oxyde de fer noir (CI: 77499) | 0,35 % | 0,35 % | 0,80 % | 0,35 % |
| Poudre de nylon 12 (ORGASOL 2002 EXD NAT COS 204 de la société ARKEMA) | 20,00 % | 20,00 % | 20,00 % | 20,00 % |
| Polydiméthylsiloxane (viscosité : 10 cst) (FLUID DC 200 10 CS de la société DOW CORNING) | 1,23% | 1,23% | 1,23% | 1,23% |
| Mélange polydiméthylsiloxane/triméthylsiloxy silicate (DOW CORNING 593 FLUID de la société DOW CORNING) | 0,39% | 0,39% | 0,39% | 0,39% |
| Polyméthylcétyl diméthylsiloxane (PM : 900 - viscosité : 15/25 cst) (ABIL WAX 9801 de la société GOLDSCHMIDT) | 0,24 % | 0,24 % | 0,24 % | 0,24 % |
| Mica (CI: 77019) | 20,00 % | 20,00 % | 30,00 % | 20,00 % |

(suite)

| | Exemple 1 | Exemple 2 | Exemple 3 | Exemple 4 |
|---|---|---|---|---|
| Citrate de tri-iso-cétyle (CITMOL 316 de la société BERNEL CHEMICAL 5ALZO) | 0,10 % | 0,10 % | 0,10 % | 0,10 % |
| Oxychlorure de bismuth (CI: 77163) | 10,00 % | 10,00 % | 0,00 % | 10,00 % |
| Talc | 36,35 % | 34,35 % | 33,40 % | 34,35 % |
| Stéarate de magnésium | 4,00 % | 4,00 % | 4,00 % | 4,00 % |
| Néopentanoate d'isodécyle désodorisé (DUB VCI 10 de la société STERINERIE DUBOIS) | 4,05 % | 4,05 % | 4,05 % | 2,05 % |
| METHOXYCINNAMATE D' ETHYLHEXYLE | 0,00% | 0,00% | 0,00% | 2,00% |

## Revendications

1. Composition cosmétique pulvérulente, **caractérisée en ce qu'**elle comprend, dans un milieu physiologiquement acceptable, une quantité efficace à titre de liant d'au moins un ester court et d'au moins un dérivé siliconé, la teneur massique en ester(s) court(s) étant supérieure à la teneur massique en dérivé(s) siliconé(s).

2. Composition cosmétique pulvérulente, **caractérisée en ce qu'**elle comprend, dans un milieu physiologiquement acceptable, une quantité efficace à titre de liant d'au moins un ester court et d'au moins un dérivé siliconé, ladite quantité étant inférieure à 8 % en poids, par rapport au poids total de la composition.

3. Composition cosmétique pulvérulente, notamment pour le maquillage de la peau, **caractérisée en ce qu'**elle comprend, dans un milieu physiologiquement acceptable au moins :

   - une quantité efficace à titre de liant d'au moins un ester court et
   - au moins 15 % en poids d'au moins une charge organique sphérique par rapport au poids total de la composition.

4. Composition cosmétique compactée comprenant, dans un milieu physiologiquement acceptable au moins :

   - une phase pulvérulente,
   - une quantité efficace à titre de liant d'au moins un ester court,

   ladite composition possédant après décompactage une granulométrie D50 exprimée en volume inférieure à 12 µm et une polydispersité inférieure à 2.

5. Composition selon la revendication 4, **caractérisée en ce que** la granulométrie D50 après décompactage est inférieure à 10,5 µm.

6. Composition cosmétique pulvérulente comprenant à titre de liant au moins un ester court, **caractérisée en ce qu'**elle est obtenue par micronisation, notamment par jet d'air, de l'ensemble de ses composants, ledit ester court étant ajouté à l'issue de ladite étape de micronisation, sous agitation mécanique, à raison d'au moins 90 % en poids par rapport à son poids total dans ladite composition.

7. Composition selon l'une quelconque des revendications 3 à 6, **caractérisée en ce qu'**elle comprend en outre, à titre de liant, au moins un dérivé siliconé.

8. Composition selon l'une quelconque des revendications 1, 2 et 7, **caractérisée en ce que** le dérivé siliconé est choisi parmi la famille des huiles siliconées, la famille des résines siliconées, la famille des cires siliconées et la famille des gommes siliconées.

9. Composition selon la revendication précédente, **caractérisée en ce qu'**elle comprend au moins deux dérivés siliconés appartenant à deux familles distinctes.

10. Composition selon la revendication 8 ou 9, **caractérisée en ce qu'**elle comprend au moins deux dérivés siliconés

appartenant à une même famille.

**11.** Composition selon l'une quelconque des revendications 1, 2 et 7 à 10, **caractérisée en ce qu'**elle contient au moins une huile de silicone choisie parmi la diméthicone, des polydiméthylsiloxanes, des polyplénylméthylsiloxane, de l'octaméthyl cyclotétrasiloxane, du décaméthyl cyclopentasiloxane, du dodécaméthyl cyclohexasiloxane, de l'heptaméthyl trisiloxane hexyl, de l'heptaméthyloctyl trisiloxane, de l'héxaméthyl disiloxane, de l'octaméthyl trisiloxane, du décaméthyl tétrasiloxane, du dodécaméthyl pentasiloxane, des phényltriméthicones, des phényldiméthicones, des phényl triméthylsiloxy diphénylsiloxanes, des diphényldiméthicones, des diphényl méthyldiphényl trisiloxanes, des 2-phényléthyl triméthylsiloxysilicates et leurs mélanges.

**12.** Composition selon l'une quelconque des revendications 1, 2 et 7 à 11, **caractérisée en ce qu'**elle contient au moins une résine de silicone choisie parmi la triméthylsiloxy silicate et ses mélanges.

**13.** Composition selon l'une quelconque des revendications 1, 2 et 7 à 12, **caractérisée en ce qu'**elle contient au moins une cire de silicone choisie parmi la polydiméthylcétyl diméthylsiloxane et ses mélanges.

**14.** Composition selon l'une quelconque des revendications 1, 2 et 7 à 13, **caractérisée en ce que** le rapport pondéral ester court/dérivé(s) siliconé(s) est supérieur à 1.

**15.** Composition selon l'une quelconque des revendications précédentes, **caractérisée en ce que** ledit ester court répond à la formule (I) suivante :

$$R_1\text{-CO-O-}R_2 \text{ (I)}$$

dans laquelle :

- $R_1$ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué, et
- $R_2$ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

**16.** Composition selon l'une quelconque des revendications précédentes, **caractérisée en ce que** les esters courts sont en $C_9$ à $C_{40}$ et notamment en $C_{10}$ à $C_{25}$ et plus particulièrement en $C_{12}$ à $C_{20}$.

**17.** Composition selon l'une quelconque des revendications précédentes, **caractérisée en ce que** l'ester court est choisi parmi le néopentanoate d'isodécyle, l'isononanoate d'isononyle, l'octanoate de cétostéaryle, le myristate d'isopropyle, le palmitate d'éthyl-2-hexyle, le stéarate d'octyl 2-dodécyle, l'érucate d'octyl 2-dodécyle, l'isostéarate d'isostéaryle, et leurs mélanges.

**18.** Composition selon l'une quelconque des revendications 1 à 2 et 4 à 17, **caractérisée en ce que** la composition comprend en outre au moins une charge organique sphérique.

**19.** Composition selon la revendication 3 ou 18, **caractérisée en ce que** la charge organique sphérique est du polyamide.

**20.** Procédé de préparation d'une poudre pulvérulente composée d'au moins une charge pulvérulente et d'une quantité efficace à titre de liant d'au moins un ester court, **caractérisé en ce qu'**il comprend au moins les étapes consistant à :

- microniser par jet d'air l'ensemble des composants de ladite charge pulvérulente en présence, le cas échéant, de moins de 10 % en poids dudit ester court par rapport à son poids total dans ladite composition, et
- mélanger la charge pulvérulente micronisée obtenue à l'issue de l'étape précédente avec au moins 90 % en poids dudit ester court par rapport à son poids total dans ladite composition.

**21.** Procédé selon la revendication précédente, **caractérisé en ce que** la totalité en ester(s) court(s) est ajoutée à l'issue de la première étape de micronisation.

**22.** Procédé de maquillage d'une matière kératinique, comprenant l'application sur la surface à maquiller d'une composition selon l'une quelconque des revendications 1 à 19.